# EUROPEAN PATENT APPLICATION

(11) **EP 4 331 581 A1**
(43) Date of publication of application: **06.03.2024**
(21) Application number: 22795409.6
(22) Date of filing: 25.03.2022
(51) Int. Cl.: A61K 31/4172, A23L 33/175, A61P 13/12

(54) **COMPOSITION FOR AMELIORATING OR SUPPRESSING DECLINE OF KIDNEY FUNCTIONS**

(30) Priority: 26.04.2021 JP 2021074321
(71) Applicant: Suntory Holdings Limited, Osaka 530-8203 (JP)
(72) Inventor: KATSUBE, Makoto, Soraku-gun, Kyoto 619-0284 (JP); WATANABE, Hiroshi, Soraku-gun, Kyoto 619-0284 (JP); MURAYAMA, Norihito, Soraku-gun, Kyoto 619-0284 (JP)
(74) Representative: Vossius & Partner Patentanwälte Rechtsanwälte mbB
(86) International application number: PCT/JP2022/014246
(87) International publication number: WO 2022/230491

(57) **Abstract**

The present invention aims to provide a composition for reducing renal function decline or ameliorating renal function and a method of reducing renal function decline or ameliorating renal function. The present invention relates to a composition for reducing renal function decline or ameliorating renal function, the composition containing L-ergothioneine or a salt thereof as an active ingredient.

## Description

### TECHNICAL FIELD

The present invention relates to a composition for reducing renal function decline or ameliorating renal function. The present invention also relates to, for example, a method of reducing renal function decline or ameliorating renal function.

### BACKGROUND ART

The kidneys are organs that filter the blood to remove urine including waste, extra water, and the like from the body, thereby maintaining body fluid homeostasis. In medical checkups and the like, the condition of renal function is determined using the blood urea nitrogen (BUN) concentration (BUN level) and the like as indices. In countries such as Japan, recent excessive salt and excessive protein diet has increased the burden on the kidneys, causing renal function decline. Renal function is known to decline with aging.

Ergothioneine is an amino acid found in mushrooms and the like. L-ergothioneine is present in nature. According to Patent Literature 1, the peritesticular fat weight and the triglyceride (neutral fat) level in plasma were lower in mice orally administered with ergothioneine than in mice not orally administered with ergothioneine.

### CITATION LIST

### - Patent Literature

Patent Literature 1: JP 2011-102286 A

### SUMMARY OF INVENTION

### - Technical Problem

However, it has not been known that L-ergothioneine or a salt thereof has an action to reduce renal function decline or ameliorate renal function. Generally, renal dysfunction is irreversible with few subjective symptoms. Thus, there has been a demand for components effective in reducing renal function decline or ameliorating renal function and applicable to foods for specified health uses, foods with function claims, pharmaceutical products, and the like.

The present invention aims to provide a composition for reducing renal function decline or ameliorating renal function. The present invention also aims to provide a method of reducing renal function decline or ameliorating renal function.

### - Solution to Problem

As a result of extensive studies to solve the problem described above, the present inventors found that L-ergothioneine has an action to reduce renal function decline or ameliorate renal function.

Specifically, the present invention relates to, but is not limited to, a composition for reducing renal function decline or ameliorating renal function, a method of reducing renal function decline or ameliorating renal function, and the like described below.
(1) A composition for reducing renal function decline or ameliorating renal function, the composition containing L-ergothioneine or a salt thereof as an active ingredient.
(2) The composition according to (1) above, wherein the composition has an action to suppress an increase in blood urea nitrogen (BUN) level or reduce blood urea nitrogen (BUN) level.
(3) The composition according to (1) or (2) above, wherein the composition has an action to suppress an increase in blood uric acid level or reduce blood uric acid level.
(4) The composition according to any one of (1) to (3) above, wherein the composition has an action to suppress an increase in blood creatinine level or reduce blood creatinine level.
(5) The composition according to any one of (1) to (4) above, wherein the composition is an oral composition.
(6) The composition according to any one of (1) to (5) above, wherein the composition is a food or beverage.
(7) The composition according to any one of (1) to (6) above, wherein an amount of L-ergothioneine or a salt thereof in the composition per adult daily intake is 2 to 50 mg in terms of L-ergothioneine.
(8) A method of reducing renal function decline or ameliorating renal function, the method including administering L-ergothioneine or a salt thereof to a subject.
(9) Use of L-ergothioneine or a salt thereof for reducing renal function decline or ameliorating renal function.

### - Advantageous Effects of Invention

The present invention can provide a composition for reducing renal function decline or ameliorating renal function. The present invention can provide a method of reducing renal function decline or ameliorating renal function.

### BRIEF DESCRIPTION OF DRAWINGS

FIG. 1 is a graph showing change in BUN level (Δ in BUN) of a test food group and a control food group.
FIG. 2 is a graph showing change in blood uric acid level (Δ in uric acid) of the test food group and the control food group.
FIG. 3 is a graph showing change in blood creatinine level (Δ in creatinine) of the test food group and the control food group.

### DESCRIPTION OF EMBODIMENTS

The composition for reducing renal function decline or ameliorating renal function of the present invention contains L-ergothioneine or a salt thereof as an active ingredient. Hereinafter, the composition for reducing renal function decline or ameliorating renal function of the present invention is sometimes simply referred to as "the composition of the present invention".

L-ergothioneine is one of the amino acids.

The salt of L-ergothioneine is not limited as long as it is a pharmacologically acceptable salt or a dietary acceptable salt, and it may be either an acid salt or a basic salt. Examples of the acid salt include inorganic acid salts such as hydrochloride, sulfate, nitrate, and phosphate, and organic acid salts such as acetate, citrate, maleate, malate, oxalate, lactate, succinate, fumarate, and propionate. Examples of the basic salt include alkali metal salts such as sodium salt and potassium salt, and alkaline earth metal salts such as calcium salt and magnesium salt.

L-ergothioneine or a salt thereof that can be used may be a chemically synthesized product or a purified extract of a natural product. L-ergothioneine is abundantly present in golden/yellow oyster mushrooms (binomial name: *Pleurotus cornucopias* var. *citrinopileatus*) belonging to the genus *Pleurotus* of the family Pleurotaceae. L-ergothioneine is also present in mushrooms such as common mushrooms (binomial name: *Agaricus bisporus*) including white mushroom, cremini mushroom, and portabella mushroom; grey oyster mushroom (binomial name: *Pleurotus ostreatus*)*,* shiitake (binomial name: *Lentinula edodes*)*,* hen-of-the-wood (binomial name: *Grifola Frondosa*)*,* reishi mushroom (binomial name: *Ganoderma lucidum*)*,* lion's mane mushroom (binomial name: *Hericium erinaceus*)*,* Yanagimatsutake (binomial name: *Agrocybe aegerita*)*,* girolle (binomial name: *Cantharellus cibarius*), porcini (binomial name: *Boletus edulis*)*,* and common morel (binomial name: *Morchella esculenta*)*.* When L-ergothioneine is obtained from a natural product, preferably, it is extracted from a golden/yellow oyster mushroom, for example. L-ergothioneine or a salt thereof can also be produced by microbial fermentation. An extract containing L-ergothioneine or a salt thereof produced by microbial fermentation or a purified product thereof may be used. L-ergothioneine can be extracted and purified from natural products by known methods. L-ergothioneine or a salt thereof may be in an isolated form.

L-ergothioneine or a salt thereof is present in natural products and food and beverages, and it is a compound that has been consumed. Thus, continuous ingestion of L-ergothioneine or a salt thereof, for example, is less likely to cause problems in terms of safety.

In the present invention, the expression "reducing renal function decline" encompasses maintaining renal function, delaying the progress of renal function decline, arresting renal function decline, and the like. The expression "ameliorating renal function" includes reduction (alleviation) in the degree of renal function decline, recovery of declined renal function, improvement in renal function, and the like. Recovery includes at least partial recovery.

Renal function can be evaluated by examining at least one (preferably all the three) selected from the group consisting of blood urea nitrogen (BUN) level, blood creatinine level, and blood uric acid level. The blood BUN level, blood creatinine level, and blood uric acid level are used as indices of renal function. A suppressed increase or a reduction in at least one of BUN level, blood creatinine level, and blood uric acid level (preferably, all of these levels) usually indicates that renal function decline is reduced or that renal function is ameliorated.

As described later in Examples, ingestion of L-ergothioneine reduced blood urea nitrogen (BUN) level, blood creatinine level, and blood uric acid level, compared to no ingestion. L-ergothioneine or a salt thereof has an action to suppress an increase in BUN level or reduce BUN level, an action to suppress an increase in blood uric acid level or reduce blood uric acid level, and an action to suppress an increase in blood creatinine level or reduce blood creatinine level. Thus, L-ergothioneine or a salt thereof has an action to reduce renal function decline or an action to ameliorate renal function. L-ergothioneine or a salt thereof can be used as an active ingredient to reduce renal function decline or ameliorate renal function.

Preferably, the composition of the present invention has an action to suppress an increase in at least one selected from the group consisting of blood urea nitrogen (BUN) level, blood uric acid level, and blood creatinine level or reduce at least one selected from the above group. More preferably, the composition of the present invention has an action to suppress an increase in blood BUN level, blood uric acid level, and blood creatinine level or reduce blood BUN level, blood uric acid level, and blood creatinine level.

In one embodiment, the composition of the present invention can be preferably used as a composition for reducing renal function decline or ameliorating renal function in humans. The composition of the present invention can be used to, for example, prevent or ameliorate a condition or disease caused by renal function decline. Examples of the condition or disease caused by renal function decline include renal dysfunction, kidney failure, glomerulonephritis, uremia, gout, and uric acid stones. In one embodiment, the composition of the present invention can be used to prevent or ameliorate renal dysfunction, kidney failure, glomerulonephritis, uremia, gout, or uric acid stones.

Preventing a condition or disease encompasses preventing the onset, delaying the onset, reducing the incidence rate, reducing the onset risk, and the like. Ameliorating a condition or disease encompasses helping a subject recover from a condition or disease, alleviating a symptom of a condition or disease, favorably changing a symptom of a condition or disease, delaying or preventing the progress of a condition or disease, and the like.

The composition of the present invention is applicable for therapeutic use (medical use) and non-therapeutic use (non-medical use). The term "non-therapeutic" is a concept that does not include medical activities, i.e., a concept that does not include methods of surgery, therapy, or diagnosis of humans.

The composition for reducing renal function decline or ameliorating renal function of the present invention can be provided as an agent or the like, for example, but it is not limited thereto. The agent can be directly provided as a composition or can be provided as a composition containing the agent. In one embodiment, the composition for reducing renal function decline or ameliorating renal function of the present invention can also be referred to as an agent for reducing renal function decline or ameliorating renal function.

In order to sufficiently obtain the effect of the present invention, preferably, the composition of the present invention is an oral composition. The oral composition may be a food or beverage, an oral pharmaceutical product, an oral quasi-pharmaceutical product, or feed, preferably a food or beverage or an oral pharmaceutical product, more preferably a food or beverage.

The composition of the present invention may contain optional additives and optional components in addition to L-ergothioneine or a salt thereof, as long as the effect of the present invention is not impaired. Such additives and components can be selected according to the composition form or the like. Those that can be generally used in food and beverages, pharmaceutical products, quasi-pharmaceutical products, feed, and the like can be used. When the composition of the present invention is provided as a food or beverage, a pharmaceutical product, a quasi-pharmaceutical product, feed, or the like, the production method is not limited, and any common method can be used for production.

For example, when the composition of the present invention is provided as a food or beverage, various types of food or beverages can be provided by adding a component usable in food and beverages (e.g., a food material or an optional food additive) to L-ergothioneine or a salt thereof. Non-limiting examples of the food or beverage include general food and beverages, health foods, health beverages, foods with function claims, foods for specified health uses, dietary supplements, and food and beverages for the sick. The health foods, foods with function claims, foods for specified health uses, dietary supplements, and the like can be used, for example, in various forms of preparations such as fine granules, tablets, granules, powders, capsules, chewable tablets, syrups, liquids, and liquid foods.

When the composition of the present invention is provided as a pharmaceutical product or a quasi-pharmaceutical product, for example, a pharmacologically acceptable carrier, an optional additive, or the like can be added to L-ergothioneine or a salt thereof to provide pharmaceutical products or quasi-pharmaceutical products in various dosage forms. Such a carrier, an additive, or the like may be any pharmacologically acceptable one that can be used in pharmaceutical products or quasi-pharmaceutical products. Examples thereof include excipients, binders, disintegrants, lubricants, antioxidants, and colorants. One or more of these can be used. The administration form of pharmaceutical products or quasi-pharmaceutical products may be oral or parenteral. Oral administration is preferred in order to obtain the effect of the present invention more sufficiently. When the composition of the present invention is provided as a pharmaceutical product or a quasi-pharmaceutical product, preferably, it is an oral pharmaceutical product or an oral quasi-pharmaceutical product. Examples of dosage forms for oral administration include liquids, tablets, powders, fine granules, granules, sugar-coated tablets, capsules, suspensions, emulsions, and chewable tablets. Examples of dosage forms for parenteral administration include injection and infusion. The pharmaceutical product and the quasi-pharmaceutical product may be for non-human animals.

When the composition of the present invention is provided as feed, L-ergothioneine or a salt thereof is simply added to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of L-ergothioneine or a salt thereof in the composition of the present invention is not limited and can be set according to the composition form and the like.

The amount of L-ergothioneine or a salt thereof in the composition of the present invention is preferably 0.0001 wt% or more, more preferably 0.001 wt% or more and is preferably 90 wt% or less, more preferably 50 wt% or less in terms of L-ergothioneine, for example. In one embodiment, the amount of L-ergothioneine or a salt thereof in the composition is preferably 0.0001 to 90 wt%, more preferably 0.001 to 50 wt% in terms of L-ergothioneine. In one embodiment, when the composition of the present invention is provided as a food or beverage, a pharmaceutical product, a quasi-pharmaceutical product, feed, or the like, preferably, the amount of L-ergothioneine or a salt thereof is in the above ranges.

In the case of L-ergothioneine, the expression "the amount in terms of L-ergothioneine" or a similar expression refers to the amount of L-ergothioneine. In the case of a salt of L-ergothioneine, the expression refers to a value obtained by multiplying the molar number of the salt by the molecular weight of L-ergothioneine.

The composition of the present invention can be fed or administered by a method appropriate to the composition form. In order to more sufficiently obtain the effect of the present invention, preferably, the composition of the present invention is orally fed (orally administered). The intake (administration amount) of the composition of the present invention is not limited as long as it is an amount that provides an effect of reducing renal function decline or ameliorating renal function. The intake may be appropriately set according to the administration form, administration method, body weight of a subject, and the like.

In one embodiment, when orally feeding or administering the composition of the present invention to a human (adult) as a subject, the intake of L-ergothioneine or a salt thereof in terms of L-ergothioneine per day is preferably 2 mg or more, more preferably 5 mg or more, still more preferably 10 mg or more, and is preferably 50 mg or less, more preferably 25 mg or less, still more preferably 20 mg or less. In one embodiment, when orally feeding or administering to a human (adult) as a subject, the intake of L-ergothioneine or a salt thereof in terms of L-ergothioneine per day is preferably 2 to 50 mg, more preferably 5 to 25 mg, still more preferably 5 to 20 mg, particularly preferably 10 to 20 mg. Preferably, the above amount of the composition is fed or administered in one or more portions per day, for example, in one portion or several portions (for example, two or three portions) per day. In one embodiment of the present invention, the composition of the present invention may be an oral composition for feeding or administering the above amount of L-ergothioneine or a salt thereof per 60 kg body weight per day to a human.

In one embodiment, when the composition of the present invention is parenterally administered to a human (adult), the administration amount of L-ergothioneine or a salt thereof in terms of L-ergothioneine per day is, for example, preferably 2 to 50 mg, more preferably 5 to 25 mg, still more preferably 5 to 20 mg, particularly preferably 10 to 20 mg.

In one embodiment, in the case of a human (adult), preferably, the above amount of L-ergothioneine or a salt thereof is fed or administered per 60 kg body weight per day.

In one embodiment, preferably, the amount of L-ergothioneine or a salt thereof in the composition of the present invention per adult daily intake is 2 to 50 mg in terms of L-ergothioneine. The amount of L-ergothioneine or a salt thereof in the composition of the present invention per adult daily intake is more preferably 5 to 25 mg, still more preferably 5 to 20 mg, particularly preferably 10 to 20 mg in terms of L-ergothioneine.

Continuous feeding or administration of L-ergothioneine or a salt thereof is likely to result in a higher effect of reducing renal function decline or ameliorating renal function. Thus, in a preferred embodiment, the composition of the present invention is continuously fed or administered. In one embodiment of the present invention, the composition of the present invention is continuously fed or administered preferably for at least one week, more preferably for at least two weeks.

The composition of the present invention may be fed or administered to any subject (subject to administration). The subject is preferably a human or non-human mammal, more preferably a human.

The composition of the present invention may be fed or administered to a subject needing or wanting to reduce renal function decline or ameliorate renal function. Examples of the subject to administration include humans needing or wanting to prevent or ameliorate a condition or disease caused by renal function decline (e.g., renal dysfunction, kidney failure, glomerulonephritis, uremia, gout, uric acid stones, and the like). In one embodiment, the subject to administration may be a subject with a condition or disease caused by renal function decline. In one embodiment, the subject to administration of the composition of the present invention may be a healthy person. The composition of the present invention can also be used on a healthy person, for example, for purposes such as preventing a condition or disease caused by renal function decline.

The composition of the present invention may be labeled with a function claim based on a reduction in renal function decline or amelioration of renal function. For example, the composition of the present invention may be labeled with one or more function claims such as "preventing renal dysfunction", "ameliorating renal function", and "preventing renal disease".

In one embodiment of the present invention, preferably, the composition of the present invention is a food or beverage labeled with such a function claim. The label may be one indicating use of the composition for obtaining the function. The label may be directly attached to the composition or may be attached to a container or a package of the composition.

The present invention encompasses the following method and use:
a method of reducing renal function decline or ameliorating renal function, the method including feeding or administering L-ergothioneine or a salt thereof to a subject; and
use of L-ergothioneine or a salt thereof for reducing renal function decline or ameliorating renal function.

Feeding or administering L-ergothioneine or a salt thereof to a subject can reduce renal function decline or ameliorate renal function. Preferably, L-ergothioneine or a salt thereof is orally fed or administered.

The method may be therapeutic or non-therapeutic. The use may be therapeutic or non-therapeutic.

In the method and use, L-ergothioneine or a salt thereof, preferred embodiments thereof, and the like are as described above for the composition for reducing renal function decline or ameliorating renal function of the present invention. In the method and use, preferably, L-ergothioneine or a salt thereof is fed or administered to a subject one or more times per day, for example, one to several times (e.g., two to three times) per day. The use is preferably for humans or non-human mammals, more preferably for humans. In one embodiment, L-ergothioneine or a salt thereof can be used to prevent or ameliorate renal dysfunction, kidney failure, glomerulonephritis, uremia, gout, or uric acid stones, for example, by reducing renal function decline or ameliorating renal function.

In the method and use, L-ergothioneine or a salt thereof is simply used in an amount (effective amount) that provides the effect of reducing renal function decline or ameliorating renal function. L-ergothioneine or a salt thereof, preferred administration amounts thereof, preferred subjects to administration, and the like are as described above for the composition for reducing renal function decline or ameliorating renal function of the present invention. L-ergothioneine or a salt thereof may be directly fed or administered or may be fed or administered as a composition containing L-ergothioneine or a salt thereof. For example, the composition of the present invention may be fed or administered.

L-ergothioneine or a salt thereof can be used to produce a food or beverage, a pharmaceutical product, a quasi-pharmaceutical product, feed, or the like for use in reducing renal function decline or ameliorating renal function. In one embodiment, the present invention also encompasses use of L-ergothioneine or a salt thereof for producing a composition for reducing renal function decline or ameliorating renal function.

The present invention also encompasses L-ergothioneine or a salt thereof for use in reducing renal function decline or ameliorating renal function.

### EXAMPLES

The present invention is described in further detail below with reference to examples, but the scope of the present invention is not limited thereto.

### <Example 1>

### (Study on evaluation of blood renal function markers in humans)

In order to evaluate the influence of supplements containing ergothioneine on blood renal function marker levels in humans, a placebo-controlled, randomized, double-blind, parallel-group study was conducted on subjects including male and female adults (46 subjects in a test food group; 46 subjects in a control food group; 92 subjects in total). In this study, one capsule containing 20 mg ergothioneine (test food) or one capsule not containing ergothioneine (control food) was fed per day for four weeks. Blood renal function marker levels of each subject were measured for pre-testing before starting the study (before starting ingestion of the test food or control food). After ingestion of the test food or control food for four weeks, blood renal function marker levels of each subject were measured again (testing on week 4). Renal function markers used for measurement were BUN, uric acid, and creatinine.

### (Food for evaluation)

Two types of food for evaluation, which were indistinguishable in appearance, flavor, and the like, were used.
· Test food: capsule containing a test substance (20 mg L-ergothioneine)
· Control food: capsule containing no test substance

Each type of food for evaluation contained raw materials such as dextrin, hydroxypropyl cellulose, carrageenan, potassium chloride, and titanium oxide, in addition to the test substance. The control food was produced using the same raw materials used in the test food, except that the test substance (L-ergothioneine) was not added.

### (BUN)

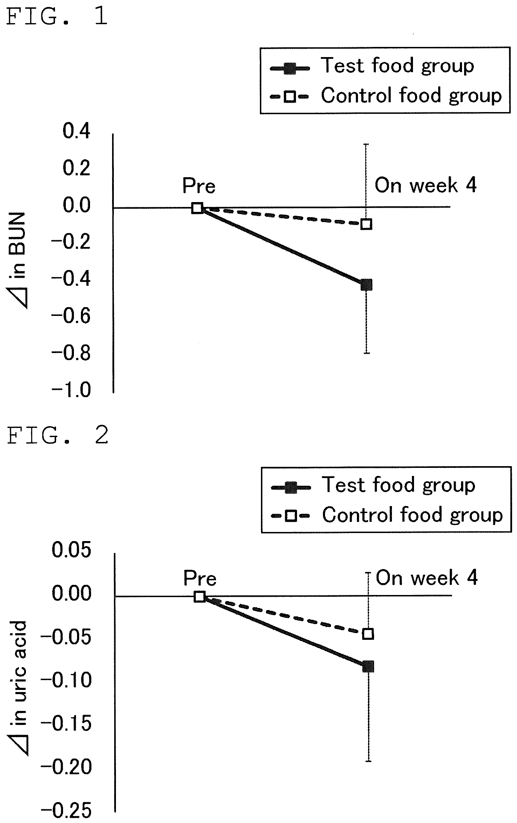

For each subject, the BUN level measured at the time of pre-testing was subtracted from the BUN level measured at the time of testing on week 4 ((level measured at the time of testing on week 4) - (level measured at the time of pre-testing)) to determine Δ in level (the amount of change in BUN level) (mg/dL). The average Δ in level of the subjects in the test food group was regarded as the amount of change in BUN (Δ in BUN) of the test food group. The average Δ in level of the subjects in the control food group was regarded as the amount of change in BUN (Δ in BUN) of the control food group. The amount of change in BUN level at the time of pre-testing was set to 0. FIG. 1 shows the results.

FIG. 1 is a graph showing change in BUN level (Δ in BUN) of the test food group and the control food group. In FIG. 1, FIG. 2, and FIG. 3, ■ (black square) indicates the test food group, and □ (white square) indicates the control food group. In FIG. 1, FIG. 2, and FIG. 3, the unit for the vertical axis is mg/dL. On the horizontal axis, "Pre" indicates at the time of pre-testing, and "On week 4" indicates at the time of testing on week 4.

According to the results, the BUN level was reduced more in the test food group than in the control food group.

### (Uric acid level)

For each subject, the uric acid level measured at the time of pre-testing was subtracted from the uric acid level measured at the time of testing on week 4 ((level measured at the time of testing on week 4) - (level measured at the time of pre-testing)) to determine Δ in level (the amount of change in uric acid level) (mg/dL). The average Δ in level of the subjects in the test food group was regarded as the amount of change in uric acid level (Δ in uric acid) of the test food group. The average Δ in level of the subjects in the control food group was regarded as the amount of change in uric acid level (Δ in uric acid) of the control food group. The amount of change in uric acid level at the time of pre-testing was set to 0. FIG. 2 shows the results.

FIG. 2 is a graph showing change in blood uric acid level (Δ in uric acid) of the test food group and the control food group. According to the results, the blood uric acid level was reduced more in the test food group than in the control food group.

### (Creatinine level)

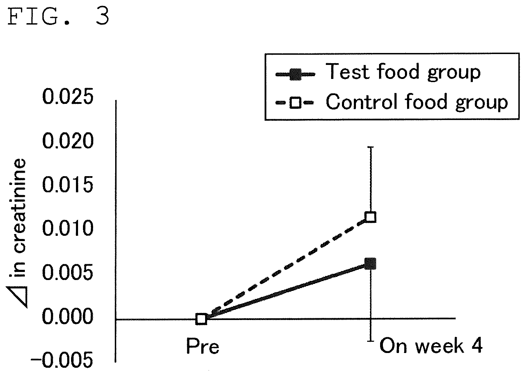

For each subject, the creatinine level measured at the time of pre-testing was subtracted from the creatinine level measured at the time of testing on week 4 ((level measured at the time of testing on week 4) - (level measured at the time of pre-testing)) to determine Δ in level (the amount of change in creatinine level) (mg/dL). The average Δ in level of the subjects in the test food group was regarded as the amount of change in creatinine level (Δ in creatinine) of the test food group. The average Δ in level of the subjects in the control food group was regarded as the amount of change in creatinine level (Δ in creatinine) of the control food group. The amount of change in creatinine level at the time of pre-testing was set to 0. FIG. 3 shows the results.

FIG. 3 is a graph showing change in blood creatinine level (Δ in creatinine) of the test food group and the control food group. According to the results, the increase in blood creatinine level was suppressed more in the test food group than in the control food group.

The above results show that L-ergothioneine or a salt thereof has an action to reduce renal function decline or ameliorate renal function.

## Claims

1. A composition for reducing renal function decline or ameliorating renal function, the composition comprising
L-ergothioneine or a salt thereof as an active ingredient.

2. The composition according to claim 1,
wherein the composition has an action to suppress an increase in blood urea nitrogen (BUN) level or reduce blood urea nitrogen (BUN) level.

3. The composition according to claim 1 or 2,
wherein the composition has an action to suppress an increase in blood uric acid level or reduce blood uric acid level.

4. The composition according to any one of claims 1 to 3,
wherein the composition has an action to suppress an increase in blood creatinine level or reduce blood creatinine level.

5. The composition according to any one of claims 1 to 4,
wherein the composition is an oral composition.

6. The composition according to any one of claims 1 to 5,
wherein the composition is a food or beverage.

7. The composition according to any one of claims 1 to 6,
wherein an amount of L-ergothioneine or a salt thereof in the composition per adult daily intake is 2 to 50 mg in terms of L-ergothioneine.

8. A method of reducing renal function decline or ameliorating renal function, the method comprising
administering L-ergothioneine or a salt thereof to a subject.

9. Use of L-ergothioneine or a salt thereof for reducing renal function decline or ameliorating renal function.
